(19) Europäisches Patentamt
European Patent Office
Office européen des brevets

(11) Publication number: 0 106 522 A1

# (12) EUROPEAN PATENT APPLICATION

(21) Application number: 83305312.7

(22) Date of filing: 12.09.83

(51) Int. Cl.³: C 07 H 3/06
C 13 K 13/00

(30) Priority: 13.09.82 US 417295

(43) Date of publication of application: 25.04.84 Bulletin 84/17

(84) Designated Contracting States: DE FR GB IT NL

(71) Applicant: CHARLES F. KETTERING FOUNDATION
5335 Far Hills Avenue
Dayton Ohio 45429(US)

(72) Inventor: Mort, Andrew James
606 Lakeshore Drive
Stillwater Oklahoma(US)

(74) Representative: Warren, Anthony Robert et al,
BARON & WARREN 18 South End Kensington
London W8 5BU(GB)

## (54) A method for producing oligosaccharides by selectively cleaving glycosidic bonds with anhydrous hydrogen.

(57) Anhydrous HF at low temperatures solvolyzes the different glycosidic linkages in a polysaccharide at widely varying rates such that oligosaccharides can be obtained from polysaccharides in higher yields than normal. The reaction is conveniently terminated and recovery of the oligosaccharide product is greaty facilitated by neutralizing excess HF with ether.

EP 0 106 522 A1

0106522

# A METHOD FOR PRODUCING OLIGOSACCHARIDES BY SELECTIVELY CLEAVING GLYCOSIDIC BONDS WITH ANHYDROUS HYDROGEN FLUORIDE

The present invention relates to a method for producing oligosaccharides and more particularly to a method for cleaving glycosidic bonds with a higher degree of selectivity.

Methods for cleaving the glycosidic linkages forming a polysaccharide are known as is the fact that the strength of these linkages and their susceptibility to hydrolysis varies depending upon the sugars involved and the character of the linkages. However, previous methods for cleaving polysaccharides have not been highly selective. There are relatively few examples of polysaccharides that can be successfully cleaved to oligosaccharides by these methods and as a result the prior methods have not been a reliable means of obtaining oligosaccharides. In the case of many polysaccharides, previous reactions nearly completely hydrolyze the polysaccharide to monosaccharides.

One technique used in the past to produce oligosaccharides is a so called partial hydrolysis where the polysaccharides are dissolved in a solution of a strong mineral acid such as sulfuric acid and heated to temperatures on the order of 180 to 200°F. This procedure produces a complex mixture of sugars including oligosaccharides and monosaccharides. This mixture can be extremely difficult to separate. In addition, the reaction is only slightly selective and product yields are low. As the reaction continues, the oligosaccharides which are formed early in the reaction are gradually hydrolyzed to monosaccharides. As a result of the

BAD ORIGINAL

difficulty in separating product and the indiscriminate reactions, large quantities of polysaccharides are required to obtain small amounts of oligosaccharide.

Thus, there is a need for a method for more selectivly cleaving glycosidic linkages in a polysaccharide than previous methods and which is capable of providing oligosaccharides in higher yields.

The foregoing need is satisified in the present invention wherein a method is provided for cleaving polysaccharides using anhydrous hydrogen fluoride.

The present invention is embodied in a method for selectively cleaving glycosidic linkages in a polysaccharide by solvolysis with anhydrous HF at low temperatures. In accordance with one embodiment of the invention, a polysaccharide is contacted with anhydrous HF in a reaction vessel. In many cases the polysaccharide is actually dissolved in the HF but in others, depending on the nature of the polysaccharide, it is not and the reaction proceeds at the interface of the liquid HF and the polysaccharide.

The reaction is generally carried out using an HF reaction apparatus in which a reaction vessel containing the polysaccharide is cooled to a temperature which is well below the boiling point of the HF and the HF is introduced into the reaction vessel by condensation of HF gas. The HF is used in a large excess making the reaction independent of its concentration. The reaction usually runs about 15 minutes or less and may be terminated by neutralizing the HF or by quenching the reaction with an inert organic solvent such as ethyl ether.

BAD ORIGINAL

Of these two techniques, the latter is preferred because the oligosaccharide yield is higher and is more easily separated. The HF can also be recovered and reused in this process.

Neutralization may also be accomplished by pouring the reactants into a slurry of a solid neutralizing agent such as calcium carbonate.

The present invention will be described in more detail by reference to the accompanying drawing wherein:

The Figure is a diagram of a typical reaction apparatus for carrying out the invention method.

The invention method is typically performed using an HF reaction apparatus such as that shown in the figure. The apparatus is indicated by the numeral 10 and comprises a horizontal manifold 12 having a series of valves 14, 16, and 18 therein. The valve 16 provides access to a mercury manometer 20. One end of the manifold 12 is connected to a cylinder 22 of compressed HF gas whereas the other end is connected via three-way valve 13 to a vacuum pump 32 and a source of compressed nitrogen 34. Both the vacuum pump 32 and the nitrogen source 34 communicate with the manifold 12 via in-line traps 26 and 27, respectively, which are filled with an agent such as CaO which removes HF from the gases that pass therethrough. The system is also provided with an air inlet, preferably upstream of trap 26, which can be used to bleed air into the system when required.

A vertical reservoir 36 is provided in line between valves 16 and 18. Reservoir 36 is optional and functions as a low volume source of HF and permits the cylinder 22 to be shut off prior to

BAD ORIGINAL

reaction, thus minimizing the danger of high volumes of HF escaping into the atmosphere in the event of a break down in the system.

The apparatus 10 is also provided with a vertical equilibration vessel 38 which is coupled to the manifold 12 via a two-way valve 40. The equilibration vessel 38 is in turn connected to a reaction vessel 42 via a piece of flexible Teflon tubing 44. The tubing 44 extends from the bottom of vessel 38 into the bottom of reaction vessel 42 and is equipped with an in-line valve 46. The reaction vessel 42 is also coupled to manifold 12 via a length of vertical tube equipped with a two way valve 48. A second piece of Teflon tubing 50 with valve 52 extends from inside the bottom of reaction vessel 42 to cooling coil 54 and from the coil to a quenching vessel 56 such as a Teflon pipe containing a neutralizing and/or quenching agent 57 such as a slurry of dry ice and a neutralizing agent such as calcium carbonate or a mixture of ether and dry ice.

The embodiment illustrated in the Figure is designed for using ether to quench the reaction. Where ether is used, the oligosaccharides precipitate from the HF solution and, as such, after the reaction has been stopped, the products are discharged through valve 58. An inline Teflon filter 60 upon which the oligosaccharides are collected as the other products are discharged into a collection vessel 62 is provided.

In the illustrated apparatus the vessels 36, 38 and 42 and the cooling coil 54 may be cooled with cooling baths (not shown). The type of bath used will depend on the temperature which is desired in the particular vessel. The cooling baths used in laboratory applications are well known. Mixtures of dry ice and various organic liquids such as acetone, chlorobenzene and carbon tetrachloride can be used.

BAD ORIGINAL

The operation of the above system is now discussed in more detail. The apparatus 10 is evacuated prior to reaction in order to remove moisture from the system. It also facilitates the operation of the system not to move the HF gas against a pressure head. For this purpose the system is equipped with vacuum pump 32 which is connected to the system via trap 26 filled with calcium oxide. Prior to evacuating the system the polysaccharide is placed in reaction vessel 42 so that upon evacuating the system any moisture present in the polysaccharide is also removed.

HF is first transferred from the cylinder 22 to reservoir 36. This is accomplished by evacuating the system by closing the valves 14 and 16 and opening the valve 18 to slowly bleed HF from the cylinder 22 into the manifold 12. The reservoir 36 is immersed in a cooling bath such as a mixture of dry ice and acetone and the HF collects by condensation therein. After a suitable quantity of HF has been collected in reservoir 36, the valve 18 can be closed and the cylinder 22 shut down.

Thereafter the HF is transferred from reservoir 36 to an equilibration vessel 38 which is used to cool the HF to the temperature which is used for the reaction. This is accomplished by opening valves 16 and 40 and cooling the vessel 38 such that the HF condenses therein. Valve 16 is then closed and the HF is retained in the equilibration vessel 38 a sufficient time to bring the HF uniformly to the reaction temperature. At the same time, vessel 42 containing the polysaccharide is equilibrated to the reaction temperature. When the HF is equilibrated, it is transferred to the reaction vessel 42 via Teflon tubing 44 which extends from the bottom of vessel 38. The transfer is effected

BAD ORIGINAL

by pressurizing the equilibration vessel 38 with dry nitrogen. This is accomplished by opening valves 14 and 40, closing valve 48, and switching valve 13 from the vacuum 32 to the nitrogen 34. By pressurizing vessel 38 and opening valve 46, liquid HF is transferred from vessel 38 to the reaction vessel 42 into which the polysaccharide has already been placed. Reaction vessel 42 is cooled to the same temperature as equilibration vessel 38. The polysaccharide must be equilibrated to the reaction temperature before the HF transfer is effected to reduce the occurrence of undesired cleavages.

The HF is allowed to react with the polysaccharide about 5 to 20 minutes. Shorter or longer reactions times may be required with some polysaccharides depending on the reaction conditions. Generally reaction times of about 10 to 15 minutes are used. Because the HF reaction of the present invention is to a certain extent time independent, the reaction time is not as critical as it is in earlier hydrolysis reactions where the oligosaccharides can rapidly hydrolyze.

Following the solvolysis reaction, the reaction products are transferred to the recovery vessel 56. This transfer is effected in the same manner as the HF transfer from vessel 38 to vessel 42. A Teflon line 50 extends from the bottom of reaction vessel 42 to the recovery vessel 56 and is equipped with a valve 52. The reaction vessel is pressurized by opening valve 48 and closing valves 16, 40 and 46.

The selectivity of the invention reaction is highly temperature dependent. As explained in more detail below small differences in solyolysis activation energy between glycosidic bonds is

BAD ORIGINAL

believed to be exploited. Hence, it is essential that the reaction products not be allowed to warm in the presence of HF to prevent the oligosaccharides thus formed from being solvolyzed further. Thus, the reaction products are not allowed to warm to above the reaction temperature until the HF has been neutralized. For this reason, it is desirable to provide a cooling coil 54 in line 50 which is cooled to approximately the same temperature or a temperature lower than the reaction vessel using a cooling bath or other refrigeration means. Similarly the neutralizing bath is cooled to the reaction temperature or below.

The reaction apparatus illustrated in the Figure is designed for terminating the reaction by quenching with an organic solvent such as ether. Product from line 50 is discharged into vessel 56 which contains ether and dry ice. The neutralizing agent (in this case ether) is cooled so that heat generated upon neutralization of the HF does not enable other glycosidic linkages to react with the HF still present thereby reducing yields. Upon contact with the ether, the oligosaccharides precipitate from the HF solution. The oligosaccharides are removed from the HF by passing the mixture through an in line Teflon filter 60 where the oligosaccharides are collected and the ether, HF and monosaccharides are discharged. Transfer of the product from vessel 56 to filter 60 is controlled with a needle valve 58.

In addition to ethyl ether, other low boiling organic solvents can be used to quench the HF reaction. The organic solvent selected must be one which neutralizes the HF, for example, by forming a complex with it, and one in which the oligosaccharides are substantially insoluble and

BAD ORIGINAL

inert. In the preferred process, the HF is recovered and reused. For such a process a solvent which complexes with the HF and which releases the HF upon heating after removal of the oligosaccharides would be desirable.

Another method of terminating the solvolysis reaction is to discharge the reaction products into a vessel containing a neutralizing agent such as calcium carbonate. To prevent solvolysis of the oligosaccharides, the neutralizing agent should be provided in a slurry of dry ice. Otherwise, as the HF is neutralized, the reaction products would heat to a point that the HF could react with linkages not solvolyzable at lower temperatures and undesired cleavages would occur.

The oligosaccharides are further separated by, for example, gel permeation chromatography.

The susceptibility of a given glycosidic linkage to solvolysis and the selectivity of the solvolysis reaction depends on the sugars bridged by the linkage as well as its stereochemistry. For example, glycosidic bonds range from those which can only be solvolyzed after standing several hours at room temperature to those which are labile at -73° in 15 minutes. In accordance with the present invention the glycosidic bonds of a polysaccharide are solvolyzed in a controlled manner, such that upon solvolysis a limited number of bonds in a polysaccharide are cleaved. In this manner oligosaccharides are produced in higher yields. The frequency with which oligosaccharides are broken down into monosaccharides is reduced.

The invention method is believed to take advantage of small differences in the activation energy required to break the various glycosidic bonds making up a polysaccharide. In general, the

BAD ORIGINAL

invention method is carried out at temperatures below 0°C and preferably below -20°C. At these temperatures significant differences in the solvolysis rate of glycosidic bonds can be established such that certain glycosidic linkages are broken with a higher frequency whereas others break to only a very limited extent. As the temperature is reduced the selectivity of the solvolysis improves. Thus, in accordance with the present invention polysaccharides can be cleaved and biologically active oligosaccharides can be obtained.

HF can be used to produce specific oligosaccharides from polysaccharides in high yield. The polysaccharide produced by R. japonicum 3Ilb 38 is composed of a repeating pentasaccharide unit. After 15 minutes in HF at -23° the polymer is cleaved to a trisaccharide and two monosaccharides. After 30 minutes at -42° the major product is the pentasaccharide repeat unit. These results indicate that HF at -23° cleaves alpha linkages of alpha-linked hexoses but not those of uronic acid or beta-linked hexoses and that at -42° there is a preferential solvolysis of one of the alpha linkages. The results with other polysaccharrides confirm the higher stability of beta hexoses to HF at -23° and in addition indicate a significant difference between the solvolysis rate of beta and alpha-linked deoxy hexoses at -42°. An added advantage of using HF to produce oligosaccharides is that the acetate esters commonly found in plants and bacterial polysaccharides are not removed during the solvolysis.

The present invention will become more clearly illustrated by the following non-limiting examples:

BAD ORIGINAL

A. Polysaccharide (101 mg) from a stationary phase culture at R. japonicum 3Ilb 138 was treated in 10 ml HF at -23°C (by cooling the reaction vessel with a dry ice/carbon tetrachloride bath). After 15 minutes, the HF solution was forced into a Teflon pipe containing 125 ml of ether which was cooled by dropping lumps of dry ice into it. A precipitate formed and was collected on a Teflon filter attached to the bottom of the pipe. The ether/HF filtrate was neutralized with calcium carbonate. The precipitate was washed with 100 ml of ether to remove residual HF, then dried under vacuum. Yield: 31 mg precipitate.

The precipitate was chromatographed on a Bio-gel P-2 column and was shown to be essentially a single component, the trisaccharide

$$\mathrm{Glc} \xrightarrow{\beta\ \ 3} \overset{\mathrm{OAc}}{\overset{\downarrow 4}{\mathrm{GalUA}}} \xrightarrow{\alpha\ \ 3} \mathrm{Man}$$

2his represents 50% of the theoretical yield since the intact polysaccharide is a repeating pentasaccharide.

The calcium carbonate used to neutralize the HF was extracted with 200 ml of water. This fraction was also chromatographed on Bio-gel P-2 and shown to contain two major components, glucose and 4-0-methyl galactose. Since there was very little mannose in any fraction other than the trisaccharide, it appears that the less than 100% yield of trisaccharide was caused by manipulative losses rather than by non-specificity of the reaction.

B. 204 mg of polysaccharide from a stationary phase culture of R.japonicum 3Ilb 138 was treated with 10 ml of HF at -40° for 30 minutes and then passed through a cooling coil at -73° into a slurry of calcium carbonate dry ice and dichloromethane. After removal of the dichloromethane, the oligosaccharides were eluted with water and then passed through a Bio-bel P-2 column. The major component eluting from the column ( 40% of recovered sugar) was the pentasaccharide.

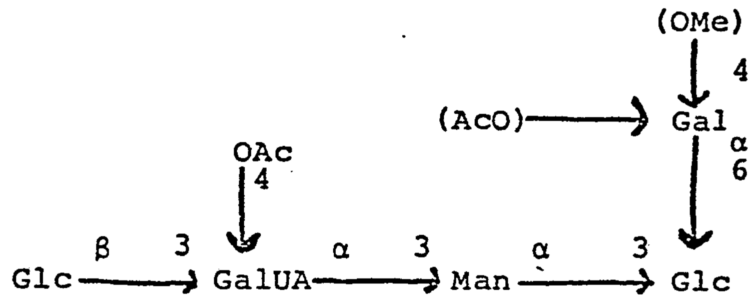

30% was probably a dimer of the repeat unit and the remainder was mostly a tetrasaccharide with a small amount of a trisaccharide identical to that produced using HF at -23°.

The polysaccharide made by R. japonicum 3Ilb 83, a 4-0-methyl glucuronorhamnan, was treated with HF at -23° for 15 minutes, neutralized with calcium carbonate, and then chromatographed on Bio-gel P-2. The material separated into two fractions: disaccharide and monosaccharide. From the structure of the polysaccharide and the composition of the fragments, the following fragmentation pattern was deduced:

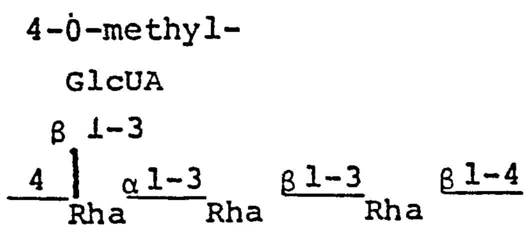

When the polysaccharide was treated at -40° and analyzed as above, three fractions were resolved on the Bio-gel P-2 column. The two major fractions corresponded to a tetrasaccharide and a disaccharide. The compositions corresponded to the tetrasaccharide having resulted from a single cleavage of the polymer per repeat unit, and the disaccharide (actually two different ones, 4-O-methylglucuronic acid rhamnose and a rhamnose disaccharide) having resulted from two breaks per repeat unit.

While the methods herein described constitute preferred embodiments of the invention, it is to be understood that the invention is not limited to these precise methods, and that changes may be made without departing from the scope of the invention, which is defined in the appended claims.

BAD ORIGINAL

CLAIMS

1. A method for producing oligosaccharides which comprises;

(a) reacting a polysaccharide with anhydrous, liquid HF at a reaction temperature less than 0°C at which temperature the glycosidic bonds forming said polysaccharide exhibit different rates of solvolysis in HF,

(b) maintaining said liquid HF in contact with said polysaccharide at said reaction temperature for a time sufficient to selectively solvolyze said polysaccharide and produce oligosaccharides,

(c) terminating said solvolysis without substantial solvolysis of the oligosaccharides produced in step (b) by quenching said reaction by the addition of an organic solvent and/or by neutralizing said HF with a neutralizing agent, and

(d) separating oligosaccharides from the reaction products.

2. The method of claim 1, wherein said reaction temperature is less than -20°C.

3. The method of claim 2, wherein said solvolysis is terminated by quenching the products of said reaction with an organic solvent which neutralizes said HF and from which oligosaccharides precipitate.

4. The method of claim 3, wherein said solvolysis is terminated by contacting the reaction products with ether.

BAD ORIGINAL

5. The method of claim 3, wherein said solvent is the same temperature as said reaction temperature or a lower temperature.

6. The method of claim 1, wherein said reaction temperature is less than -40°C.

7. The method of claim 1, wherein said organic solvent and/or said neutralizing agent is added to said reaction as a slurry with dry ice.

8. The method of claim 1, wherein said organic solvent is ethyl ether.

9. The method of claim 8 wherein said neutralizing agent is calcium carbonate.

BAD ORIGINAL

10. A method for selectively cleaving the polysaccharide produced by R.Japonicum 3I 1b138 which comprises:

(a) reacting the polysaccharide produced by R.Japonicum 3I 1b138 with anhyarous, liquid HF at a reaction temperature less than 0°C, at which temperature the glycosidic bonds forming said polysaccharide exhibit different rates of solvolysis in HF,

(b) maintaining said liquid HF in contact with said polysaccharide at said reaction temperature for a time sufficient to selectively solvolyze said polysaccharide and produce oligosaccharides,

(c) terminating said reaction without substantial solvolysis of the oligosaccharides produced in step (b) by quenching said reaction with the addition of an organic solvent and/or by neutralizing said HF, and

(d) separating said oligosaccharides from the reaction products.

11. The method of claim 10, wherein said reaction temperature is less than -20°C.

12. The method of claim 11, wherein said solvolysis reaction is terminated by quenching with an organic solvent which neutralizes said HF and from which said oligosaccharides precipitate.

13. The method of claim 12, wherein said solvolysis is terminated by contacting the reaction products with ethyl ether.

BAD ORIGINAL

14. The method of claim 10, wherein said organic solvent and/or said neutralizing agent is added to said reaction products as a slurry in dry ice.

15. The method of claim 10, wherein said reaction temperature is less than -40°C.

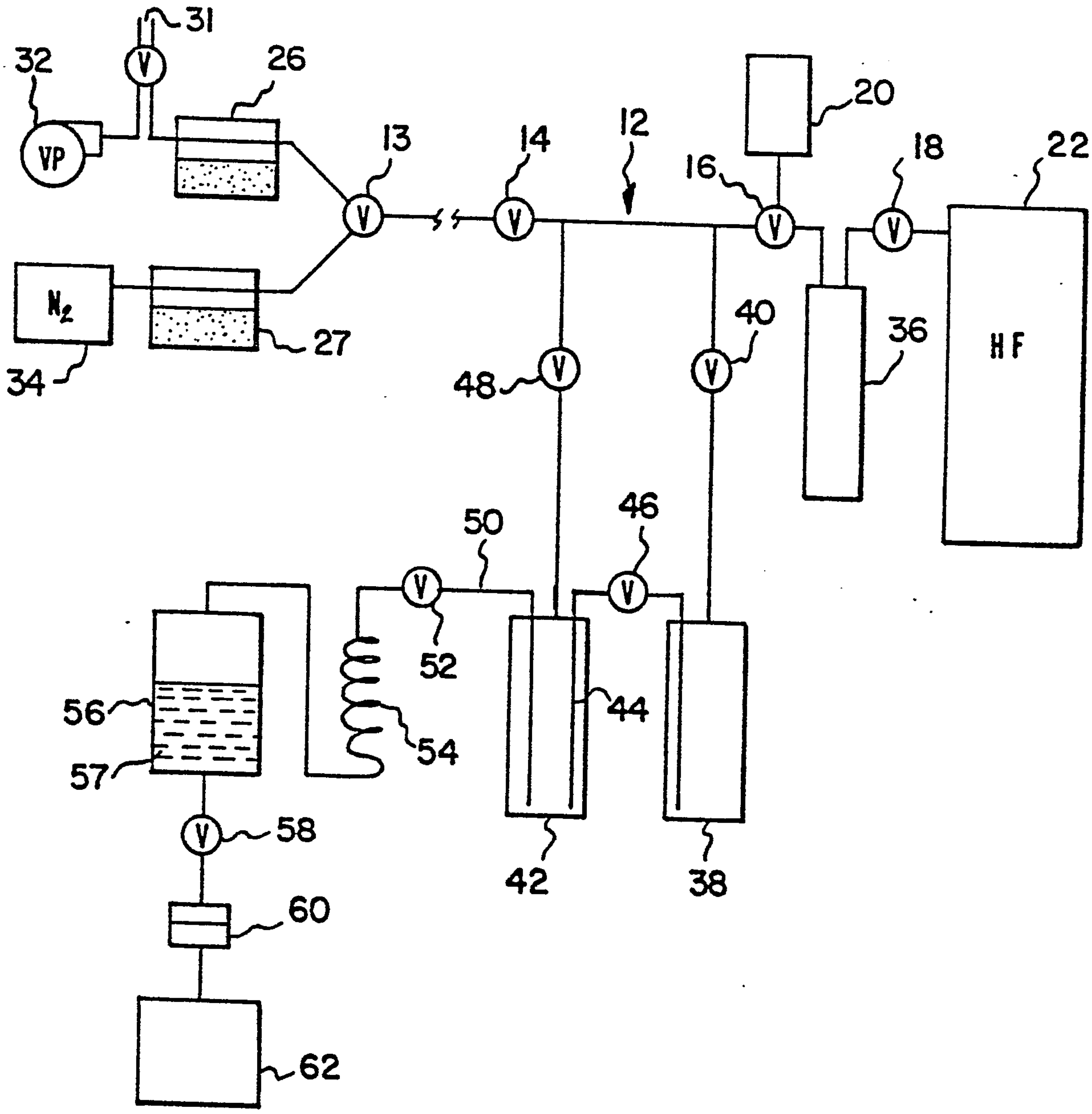
31
32
26
VP
13
14
12
20
16
18
22
N2
27
34
40
36
HF
48
50
46
52
44
56
54
57
58
42
38
60
62

0106522

European Patent Office

# EUROPEAN SEARCH REPORT

Application number

EP 83 30 5312

## DOCUMENTS CONSIDERED TO BE RELEVANT

| Category | Citation of document with indication, where appropriate, of relevant passages | Relevant to claim | CLASSIFICATION OF THE APPLICATION (Int Cl. 3) |
|---|---|---|---|
| X | ABSTRACTS OF PAPERS, American Chemical Society, March 29-April 3, 1981, Atlanta, Georgia, US * CARB 49 * --- | 1-15 | C 07 H 3/06<br>C 13 K 13/00 |
| X | THE JOURNAL OF BIOLOGICAL CHEMISTRY, vol. 257, no. 4, February 25, 1982, pages 1870-1875, US A.J. MORT et al.: "Application of two new methods for cleavage of polysaccharides into specific oligosaccharide fragments" ----- | 1-15 | |
| | | | TECHNICAL FIELDS SEARCHED (Int. Cl. 3) |
| | | | C 07 H 3/00<br>C 13 K 13/00<br>C 13 K 1/00 |

The present search report has been drawn up for all claims

| Place of search | Date of completion of the search | Examiner |
|---|---|---|
| THE HAGUE | 15-12-1983 | VERHULST W. |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

EPO Form 1503. 03.82